(19) Europäisches Patentamt
European Patent Office
Office européen des brevets

(11) **EP 3 082 942 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **14815239.0**

(22) Anmeldetag: **17.12.2014**

(51) Int Cl.:
*A61K 38/22* *(2006.01)*          *A61K 38/27* *(2006.01)*
*A61K 38/28* *(2006.01)*          *A61K 38/36* *(2006.01)*
*A61M 37/00* *(2006.01)*          *A61K 31/27* *(2006.01)*
*A61N 1/04* *(2006.01)*          *A61K 31/4468* *(2006.01)*
*A61K 31/485* *(2006.01)*          *A61N 1/30* *(2006.01)*
*A61K 31/55* *(2006.01)*

(86) Internationale Anmeldenummer:
**PCT/EP2014/003399**

(87) Internationale Veröffentlichungsnummer:
**WO 2015/090583 (25.06.2015 Gazette 2015/25)**

(54) **SYSTEM FÜR DIE TRANSDERMALE ABGABE VON WIRKSTOFF**

SYSTEM FOR THE TRANSDERMAL DELIVERY OF AN ACTIVE SUBSTANCE

SYSTÈME DE DISTRIBUTION TRANSDERMIQUE DE SUBSTANCES ACTIVES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2013 EP 13199189**

(43) Veröffentlichungstag der Anmeldung:
**26.10.2016 Patentblatt 2016/43**

(73) Patentinhaber: **LTS Lohmann Therapie-Systeme AG**
**56626 Andernach (DE)**

(72) Erfinder:
• **SAMETI, Mohammad**
**53177 Bonn (DE)**
• **HACKBARTH, Ronald**
**56075 Koblenz (DE)**
• **SCHUMANN, Klaus**
**56567 Neuwied (DE)**
• **SCHMITZ, Christoph**
**56598 Rheinbrohl (DE)**

(74) Vertreter: **Schweitzer, Klaus**
**Plate Schweitzer Zounek**
**Patentanwälte**
**Rheingaustrasse 196**
**65203 Wiesbaden (DE)**

(56) Entgegenhaltungen:
WO-A1-2010/027468          WO-A1-2012/071175
US-A- 5 645 526          US-A- 5 817 044
US-A1- 2002 019 652          US-A1- 2010 076 387

**Beschreibung**

Die vorliegende Erfindung betrifft ein neues System für die Abgabe von pharmazeutischen Wirkstoffen in therapeutisch wirksamer Menge an den Organismus. Vorzugsweise betrifft die vorliegende Erfindung ein einfach zu handhabendes System für die transdermale Abgabe von in Flüssigkeit gelöstem, pharmazeutischem Wirkstoff in therapeutisch wirksamer Menge, besonders bevorzugt die transdermale Abgabe von kationischem Wirkstoff mittels Iontophorese.

Der transdermale Weg der parenteralen Verabreichung bietet zahlreiche Vorteile gegenüber anderen Verabreichungswegen. Verfahren und Systeme zur Verabreichung von Arzneimitteln durch die Haut sind auf dem Gebiet der Pharmazie weitgehend bekannt. Typischerweise erfolgt die transdermale Verabreichung mit passiven transdermalen Systemen (z.B. Transdermale, Therapeutische Systeme, TTS), bei denen pharmazeutische Wirkstoffe in definierten Mengen mittels Diffusion durch die Haut dem Organismus zugeführt werden.

Speziell bei der transdermalen Zufuhr von in Flüssigkeit gelösten Wirkstoffen gibt es das Problem, dass ein wirkstoffhaltiges Trägermaterial aus Vlies oder aus Schwammtuch oder ein Gel getrennt von der Deckschicht, mit der zusammen der Wirkstoff im TTS an der Haut fixiert wird, aufbewahrt werden muss, weil sonst die Lagerstabilität über eine längere Zeitdauer hinweg nicht gewährleistet ist oder weil ggf. der Wirkstoff gekühlt aufbewahrt werden muss oder weil er ggf. empfindlich ist gegenüber Oxidation. Der Anwender eines medizinischen Pflasters muss dann aus einer vorher durch Entfernen der Verschlussfolie zu öffnenden, meist versiegelten und damit flüssigkeitsdichten Verpackung das wirkstoffhaltige Trägermaterial auf die Deckschicht eines transdermalen therapeutischen Systems (TTS) übertragen und es darauf fixieren. Der Anwender soll dabei aber wegen der Gefahr der Keim Übertragung das wirkstoffhaltige Trägermaterial möglichst nicht mit den Fingern berühren und möglichst auch sonst keine zusätzlichen Hilfsmittel zum Übertragen verwenden.

Üblicherweise legt man daher die Deckschicht des TTS mit ihrer Unterseite auf die oberseitig geöffnete Verpackung eines wirkstoffhaltigen Trägermaterials und drückt dabei das Trägermaterial an die Deckschicht des TTS. Wenn das TTS danach wieder von der geöffneten Verpackung entfernt wird, bleibt das wirkstoffhaltige Trägermaterial an der Deckschicht des TTS haften und bildet zusammen mit diesem das TTS im eigentlichen Sinne, was aber nicht immer zuverlässig passiert. Oft oder zumindest mitunter kommt es vor, dass das Trägermaterial mit dem Wirkstoff einfach in der Verpackung liegen bleibt.

Unter anderem tritt dieses Problem beim Verfahren der Iontophorese auf, die dann zum Einsatz kommt, wenn die passive transdermale Arzneimittelzufuhr für bestimmte Arten von Arzneistoffen nur sehr ineffizient ist. Insbesondere sind ionisierte Medikamente oft nicht in der Lage, passiv die Haut in therapeutisch wirksamer Menge zu durchdringen.

Der Prozess der Iontophorese wurde ursprünglich von LeDuc im Jahr 1908 beschrieben, und noch früher in US-222,276 (1879) und US-486,902 (1892). Seitdem hat die Iontophorese kommerziellen Einsatz bei der transdermalen Applikation von ionisch geladenen therapeutischen Wirkstoffmolekülen wie Pilocarpin, Lidocain, Dexamethason, Lidocain und Fentanyl gefunden.

Im Allgemeinen ist die Iontophorese ein Applikationsverfahren, das auf dem Grundprinzip beruht, dass die Anwendung von elektrischem Strom externe Energie zur Verfügung stellt, mit deren Hilfe eine Erhöhung der Arzneimittelpermeabilität durch die Membranen der Haut möglich wird, was einen verbesserten Durchtritt von Wirkstoffionen durch die Haut bewirkt.

Wenn Ionen, die eine positive Ladung tragen (z. B. kationische Wirkstoffe), in oder unter der Anode eines iontophoretischen Systems platziert werden, dann wird auf diese Ionen - bei Anwendung von Strom - ein Impuls ausgeübt, der sie von der Anode weg und in Richtung des elektrischen Feldes hin zur Kathode schiebt, die in unmittelbarer Nähe der Haut angeordnet ist. Während dieses Vorgangs wird der Transport des kationischen Arzneimittels durch die Haut verbessert oder erleichtert.

Iontophorese kann mit verschiedenen Formen von pharmazeutischen Wirkstoffen durchgeführt werden, am günstigsten mit solchen, die eine elektrische Ladung besitzen und die somit im elektrischen Feld die Fähigkeit entwickeln, Barrieren (z.B. die Haut) zu überwinden.

Ein typisches iontophoretisches Arzneimittelabgabesystem umfasst ein elektrolytisches elektrisches System aus einer Anode und einer Kathode, die an verschiedenen - vorzugsweise benachbarten - Hautbereichen eines Patienten angeordnet sind, wobei jede Elektrode über einen Draht an eine externe Stromversorgung angeschlossen ist. In der Regel handelt es sich dabei um ein durch einen Mikroprozessor gesteuertes elektrisches Gerät. Solche Arten von Vorrichtungen sind bekannt, einschließlich der Systeme mit ausgesprochen einfacher Konstruktion (z.B. US 5,685,837 oder US 6,745,071) oder auch komplexere Systeme, die dem Fachmann grundsätzlich bekannt sind. Iontophoretische transdermale Systeme für Lidocain und Fentanyl wurden bereits erfolgreich in den USA am Markt eingeführt. Eine ganz besonders ausführliche Beschreibung eines Systems zur Abgabe von Arzneimittel mit Hilfe von Iontophorese findet sich in der WO 2012/071175.

US 5,558,633 beschreibt, dass für die Abgabe von Medikamenten aus Flüssigkeit oder aus gelierten wässrigen Formulierungen Iontophoresevorrichtungen besonders geeignet sind. Jedoch kann in solchen Vorrichtungen die iontophoretische Verabreichung von pharmazeutischen Wirkstoffen durch die Anwesenheit von "Hintergrund"-Elektrolyten (siehe, zum Beispiel, Luzardo-Alvarez, A., et al., Proceedings of the International Symposium on Controlled Release of bioactive Materials (2000), 27th Ed., S. 159 bis 160) stark beeinträchtigt sein. Hinsichtlich der Konstruktion von iontophoretischen Vorrichtungen besteht zudem ein Mangel an pharmazeutischen Gelen oder Flüssigkeiten, die sich selbst nicht als "Hintergrund"-Gegenionen störend auswirken.

Trotz immer noch bestehender diverser Mängel hat sich die Iontophorese als Applikationsverfahren in allen den Fällen bewährt, in denen ein herkömmliches TTS nicht ausreichend ist, schnell die Verabreichung einer therapeutisch wirksamen Dosis eines solchen Wirkstoffs zu gewährleisten. Allerdings besitzt die Iontophorese die inhärente Gefahr, dass Nebenwirkungen wie Hautreizungen, Hautrötungen, Brennen oder auch Haut Nekrose, insbesondere dann auftreten können, wenn die Stromstärke erhöht oder wenn die Iontophorese über einen längeren Zeitraum eingesetzt wird. Andererseits kann eine Erhöhung der Stromstärke für die Verabreichung von höheren Dosen von therapeutischem Wirkstoff durchaus erwünscht sein, weil die Menge der Ionen, die transportiert werden, direkt proportional ist zu der Menge des fließenden Stroms pro Zeiteinheit.

In Anbetracht der vorstehenden Ausführungen war es daher die Aufgabe der vorliegenden Erfindung, ein Verfahren zu finden, mit dem ein wirkstoffhaltiges Trägermaterial zuverlässig aus seiner Verpackung entnommen und an der Deckschicht des TTS zwecks nachfolgender Applikation, sei es mit oder ohne Unterstützung durch Iontophorese, fixiert werden kann, ohne dass dazu eine Berührung des wirkstoffhaltigen Trägermaterials mit der Hand oder ein zusätzliches Hilfsmittel zum übertragen notwendig ist.

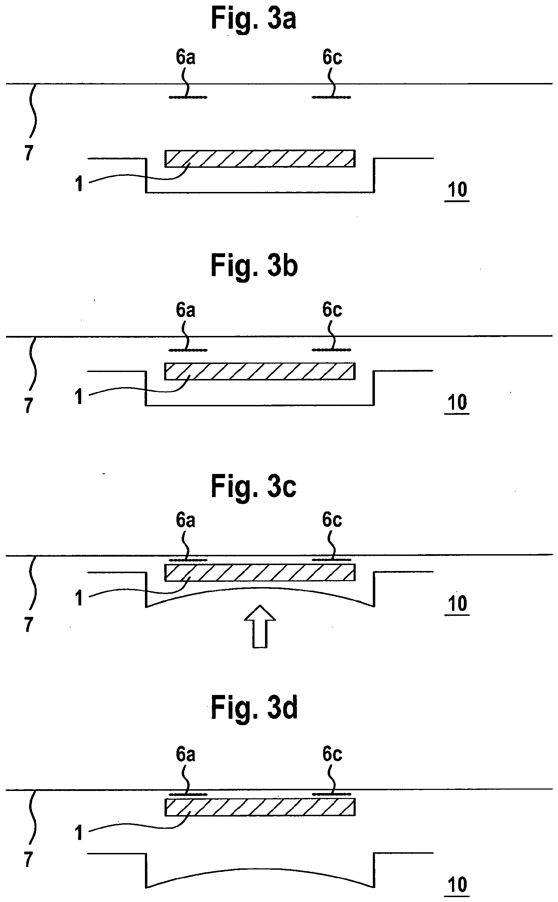

In der US 2002/0019652 A1 ist ein Pflaster zur transkutanen elektrischen Nervenstimulation (TENS) offenbart. Das TENS-Verfahren stellt ein nicht-invasives Verfahren zur Schmerzbehandlung dar, das im Prinzip ohne Zufuhr von pharmazeutischen Wirkstoffen auskommt. In einer Ausführungsform umfasst das Pflaster einen wiederverwendbaren oberen Teil, der ein Elektronik-Modul und Schalter zum Ein- und Ausschalten der Stromzufuhr sowie zur Regelung der Stromstärke enthält. Der untere Teil ist in drei nebeneinander angeordnete Abschnitte unterteilt. Die beiden äußeren Abschnitte dienen als Plus- bzw. Minus-Elektrode und sind für den direkten Hautkontakt vorgesehen, der mittlere Teil besteht bevorzugt aus sterilem Gaze-Material. Das Gaze-Material kann einen Wirkstoff enthalten. In einer besonderen Ausführungsform enthält der obere Teil, wie in Fig. 3 dargestellt, eine Schicht (70) aus einem magnetischen Polymer. Der untere Teil (40) enthält an der Oberseite ebenfalls eine Schicht (86) aus magnetischem Polymer, wie in Fig. 4 dargestellt. Die magnetischen Schichten der beiden Teile können dann aneinander haften. Gemäß Abs. [0023] wirkt die sterile Gaze in dem Bereich (80) absorbierend. Sie soll also Wundsekret oder ähnliches aufnehmen. In einer weiteren Ausführungsform sind der obere und der untere Teil durch einen Klettverschluss (Velcro) verbunden. Die magnetische Schicht (70) des oberen Teils ist dann durch eine Hälfte des Klettverschlusses ersetzt, die magnetische Schicht (86) des unteren Teils durch die andere Hälfte.

In der WO 2012/071175 A1 ist ein Iontophorese-Pflaster offenbart, das sich in einer zweiteiligen Schutzhülle befindet, Die Elektroden (22) bzw. (24) sind dabei von dem wirkstoffhaltigen Trägermaterial (42) bzw. (44) durch eine Barrierefolie (52) bzw. (54) getrennt. Die Enden (52a) bzw. (54a) der Folie ragen aus der Schutzhülle (60a) und (60b) heraus. Vor der Anwendung wird die Barrierefolie herausgezogen. Durch mechanischen Druck auf die Schutzhülle werden die Elektroden in direkten Kontakt mit dem wirkstoffhaltigen Trägermaterial gebracht. Vor dem Aufbringen des Iontophorese-Pflasters wird die Schutzhülle entfernt (s. Fig. 5A bis 5F). Durch die Trennung der Elektroden von dem wirkstoffhaltigen Trägermaterial soll das Pflaster länger lagerfähig sein.

Gelöst wird diese Aufgabe durch ein TTS gemäß des Patentanspruchs 1.

Vorteilhafte Ausgestaltungen des TTS gehen aus den Unteransprüchen hervor.

Unter einem Klettband ist anmeldungsgemäß ein textiles, beliebig oft zu lösendes Verschlussmittel zu verstehen, das auf dem Prinzip von Klettfrüchten beruht. Die bionische Umsetzung besteht in der typischen Form aus zwei gewebten Faserstreifen, von denen der eine flexible Widerhäkchen oder Pilzköpfe, der andere hingegen Schlaufen aufweist. Zusammengepresst ergeben sie einen mehr oder weniger belastungsfähigen, auf jeden Fall aber reversiblen Schnellverschluss. Gewebte Klettbänder bestehen aus Polyamid-, Polyester- oder Polyolefinfasern. Bei den Hakenbändern werden die Haken während des Webens oder später eingearbeitet. Klettbänder und Druckverschlüsse können auch mit Haftklebstoffen auf der Rückseite selbstklebend beschichtet werden.

Erfindungsgemäß bildet das Klettband, das an der Deckschicht des TTS angeordnet ist, die Hakenseite, während das wirkstoffhaltige Trägermaterial die Funktion der Schlaufenseite übernimmt. Durch Andrücken des Halteelements, das an der Deckschicht des TTS angebracht ist, auf die Oberseite der nach oben hin durch vorheriges Entfernen der Verschlussfolie geöffneten Verpackung, in der sich das wirkstoffhaltige Trägermaterial befindet, wird erfindungsgemäß bewirkt, dass sich das Klettband mit dem Trägermaterial verhakt, beim Abnehmen des TTS von der Verpackung am Halteelement des TTS haftet und nicht in der Verpackung zurückbleibt, wenn diese danach wieder entfernt wird.

Ganz allgemein gilt, dass das wirkstoffhaltige Trägermaterial sich in der Verpackung befindet, die unmittelbar vor der Übertragung des Trägermaterials plus Wirkstoff auf die Deckschicht des TTS durch Entfernen der Verschlussfolie geöffnet wird.

Dadurch, dass das wirkstoffhaltige Trägermaterial mit einer Flüssigkeit getränkt ist, haftet es an der Verpackungsfolie. Man benötigt daher eine Kraft ($x_1$) um das Trägermaterial von der Verpackungsfolie abzulösen.

Wenn nun das Trägermaterial auf das TTS übertragen werden soll, ohne dabei zusätzliche Hilfsmittel wie z.B. Finger, oder Greifelemente zu verwenden, dann wird das TTS flächig auf das Trägermaterial gelegt und angedrückt. Dabei haftet das flüssigkeitsgetränkte Trägermaterial zusätzlich an dem angedrückten TTS (Kunststofffolie/Polyesterfolie). Die Haftkräfte am TTS (=$F_{tts}$) sind dabei aber ähnlich stark wie die Haftkräfte an der Verpackung (=$F_{verp}$). Wenn das TTS nach dem Andrücken wieder entfernt wird, ist es eine Frage der Größe beider Haftkräfte, ob das wirkstoffhaltige Trägermaterial am TTS oder alternativ an der Verpackung haften bleibt. Wenn die Haftkraft am TTS ($F_{tts}$) nicht deutlich größer ist als die Haftkraft an der Verpackungsfolie ($F_{verp}$), dann besteht die Gefahr, dass das flüssigkeitsgetränkte Trägermaterial nicht wie gewünscht am TTS haftet, sondern in der Verpackung zurück bleibt.

Damit die Übertragung des wirkstoffhaltigen Trägermaterials von der Verpackungsfolie auf das TTS zuverlässig gelingt, müssen die Haftkräfte so verändert werden, dass diese deutlich unterschiedlich sind:

$$F_{tts} \gg F_{verp}$$

Die Haftkraft des wirkstoffhaltigen Trägermaterials an der Verpackungsfolie kann kaum verringert werden. Selbst Antihaftbeschichtungen verringern diese Haftkraft wegen der Viskosität der Flüssigkeit nur unwesentlich. Auch eine Profilierung der Oberfläche zeigt keine Veränderung der Haftkräfte, weil die Flüssigkeit die Oberflächenprofilierung ausfüllt und Lufteinschlüsse verdrängt.

Deshalb bietet sich zur Lösung des Problems die Erhöhung der Haftkräfte am TTS ($F_{tts}$) an, um den Übertragungsprozess zuverlässig zu gewährleisten.

Zur Erhöhung der Haftkräfte am TTS ($F_{tts}$) kommt erfindungsgemäß ein Klettband zum Einsatz. Durch das Klettband erhöhen sich die Haftkräfte des wirkstoffhaltigen Trägermaterials ($F_{tts}$) signifikant. Klettbänder haben nämlich auch die Fähigkeit, in feuchtem Zustand zu funktionieren. Beim Andrücken des wirkstoffhaltigen Trägermaterials gegen das Klettband verhaken sich die Fasern am Trägermaterial mit den Haken des Klettbands. So wird auch in feuchtem Milieu durch das Klettband die Haftkraft ($F_{tts}$) deutlich erhöht und die Übertragung des flüssigkeitsgetränkten Trägermaterials von der Verpackung auf die Deckschicht des TTS wird garantiert.

Als Deckschicht wird anmeldungsgemäß ein im wesentlichen selbsttragendes Flächengebilde bezeichnet, das die Abdeckung der Stelle auf der Haut bewirkt, an der die transdermale Abgabe von pharmazeutischem Wirkstoff an den Organismus stattfindet. Wenn die Befestigung des TTS auf der Haut nicht über ein haftklebend ausgerüstetes Überpflaster erfolgt, dann hat die Deckschicht zweckmäßigerweise auch die Funktion, das System auf der Haut zu fixieren, was durch eine flächig haftklebende oder musterförmig haftklebende Ausgestaltung der in die Richtung der Haut weisenden Seite der Deckschicht geschieht, wobei die Fläche oder die Flächen für das wirkstoffhaltiges Trägermaterial oder die wirkstoffhaltigen Trägermaterialien zumindest frei von Haftkleber sind.

In einer weiteren Ausgestaltungsform der Erfindung handelt es sich bei der Deckschicht um eine okklusive Deckschicht. Unter einer okklusiven Deckschicht ist insbesondere eine Barriereschicht für Flüssigkeiten und Wasserdampf zu verstehen, mit der verhindert wird, dass Flüssigkeit oder Wasserdampf durch die Deckschicht hindurch nach außen entweicht, wodurch das System austrocknen würde, falls es sich um die Abgabe von flüssigem oder in Flüssigkeit gelöstem pharmazeutischen Wirkstoff handelt. Die okklusive Deckschicht kann erfindungsgemäß aus einer Folie aus Kunststoff wie Polyethylen oder Polypropylen oder Polyester oder Polyurethan oder Polyamid bestehen, deren wesentliche Eigenschaft darin zu sehen ist, dass sie eine ausreichend zuverlässige Sperre für Wasser, Wasserdampf oder mit Wasser vermischten organischen Lösemitteln darstellt. Geeignete Folien aus den genannten Kunststoffen haben eine Dicke im Bereich von 5 bis 300 $\mu$m, vorzugsweise von 10 bis 200 $\mu$m, besonders bevorzugt von 12 bis 150 $\mu$m.

Die okklusive Deckschicht kann zusätzlich zur Verstärkung noch eine nicht okklusive Trägerschicht enthalten oder mit einer nicht okklusiven Trägerschicht verbunden sein. Als nicht okklusive Trägerschicht kann ein textiles Gewebe geeignet sein oder ein Vlies oder Filzmaterial oder sonstiges cellulosehaltiges Material. Die Verbindung der nicht okklusiven Trägerschicht mit der okklusiven Deckschicht kann durch Laminieren unter Druck oder durch Extrudieren oder durch Verkleben mit einem geeigneten Klebstoff erfolgen. Geeignete Kleber können aus Polyisopren oder Polyisobutylen oder Polyacrylester oder auch aus Polysiloxancopolymeren bestehen.

Auch die okklusive Deckschicht kann zusätzlich an ihrer Unterseite, das ist die in Richtung zur Haut weisende Seite, mit einer selbstklebenden Schicht versehen sein. Als selbstklebende Materialien eignen sich die vorher genannten Kleber, vorzugsweise Acrylkleber.

In einer weiteren Ausführungsform ist das wirkstoffhaltige Trägermaterial ein mit pulverförmigem Wirkstoff bestäubtes oder ein flüssigkeitsgetränktes Trägermaterial oder ein Hydrogel. Als Flüssigkeit kann erfindungsgemäß eine Lösung eines Wirkstoffes in einem Lösemittel, vorzugsweise in einem wässrigen Lösemittel, gelten oder aber auch eine Dispersion oder eine Emulsion von Wirkstoff in einem geeigneten Dispergier- oder Emulgiermittel. Als Trägermaterialien kommen dabei vliesartige Materialien oder textile gewebte Materialien oder Gewirke oder schwammförmige Materialien vorteilhaft zum Einsatz oder ggf. auch gelbildende Polymere.

Erfindungsgemäß ist die Form des länglich geformten Klettbandes der Form des wirkstoffhaltigen Trägermaterials angepasst. Wenn das wirkstoffhaltige Trägermaterial eine rechteckige Form besitzt, dann entspricht die Länge des länglich geformten Klettbandes vorzugsweise der Länge einer der Seiten des rechteckig geformten, wirkstoffhaltigen Trägermaterials. Wenn das wirkstoffhaltige Trägermaterial eine runde oder ovale Form besitzt, dann ist die Form des länglich geformten Klettbandes vorzugsweise die eines gebogenen Kreissegments mit einem Radius entsprechend dem Radius des runden oder ovalen wirkstoffhaltigen Trägermaterials.

Erfindungsgemäß geeignete Klettbänder sind selbstklebend, d.h. sie haften mit ihrer selbstklebenden Rückseite an der Deckschicht. Es ist aber auch möglich, dass ein Klettband mit einem geeigneten Kleber, z.B. einem Acrylkleber oder einem Zweikomponentenkleber oder einem Heißschmelzkleber an der Deckschicht befestigt wird.

Die Breite des länglich geformten Klettbandes kann in weiten Grenzen variieren. Das Klettband muss nicht die gesamte Fläche bedecken, über die sich das wirkstoffhaltige Trägermaterial im TTS erstreckt, sondern es ist ausreichend, wenn das länglich geformte Klettband, unabhängig von der Geometrie des wirkstoffhaltigen Trägermaterials, nur einen Teil der Fläche bedeckt. Möglich ist dabei auch, dass sich das Klettband nur in den seitenrandnahen Bereichen befindet oder dass es als umlaufender Streifen ausgebildet ist mit einer Breite im Bereich von 2 bis 15 mm, vorzugsweise von 3 bis 10 mm.

Erfindungsgemäß kann es sich auch um mehrere Klettbänder handeln, die z.B. an gegenüberliegenden Seiten der Fläche angeordnet sind, an der das wirkstoffhaltige Trägermaterial an dem TTS befestigt ist oder werden soll.

Die Erfindung betrifft auch ein Verfahren zum Befestigen eines wirkstoffhaltigen Trägermaterials an einer Deckschicht eines TTS in Anwesenheit von Klettbandsegmenten. Dabei wird das wirkstoffhaltige Trägermaterial in einer nach oben hin durch vorheriges abziehen einer Verschlussfolie geöffneten Verpackung an die okklusive Deckschicht und die dort angeordneten Klettbandsegmente angenähert. Dann wird die Verpackung zusammen mit dem darin befindlichen wirkstoffhaltigen Trägermaterial durch Pressen an die Klettbandsegmente an der okklusiven Deckschicht angedrückt. Danach kann die Verpackung losgelöst und entfernt werden, wobei das wirkstoffhaltige Trägermaterial an den Klettbandsegmenten und an der okklusiven Deckschicht fixiert bleibt.

Die Erfindung betrifft ferner die Verwendung eines Klettbandes als Bestandteil eines transdermalen therapeutischen Systems (TTS) oder als Bestandteil eines iontophoretischen transdermalen therapeutischen Systems.

Das System der vorliegenden Erfindung ist insbesondere in Verbindung mit therapeutischen Wirkstoffen, die eine kationische Struktur besitzen, insbesondere Wirkstoffe, die Amino- oder Iminogruppen in ihrem Molekül aufweisen, besonders nützlich.

Dementsprechend ist die vorliegende Erfindung geeignet für die transdermale, insbesondere die iontophoretische Verabreichung von Analgetika wie Fentanyl oder Morphin, Antiemetika wie Granisetron oder andere auf das zentrale Nervensystem wirkende Arzneistoffe wie Rivastigmin oder Galantamin.

Wenn das erfindungsgemäße System zur transdermalen Verabreichung von solchen Wirkstoffen verwendet wird, dient das flüssigkeitsgetränkte Trägermaterial als eine Matrix oder ein Reservoir, aus dem die kationischen Wirkstoffe an die Haut abgegeben werden und dann die Haut durchdringen, entweder passiv oder unterstützt durch Iontophorese.

Unter Wirkstoffen mit kationischer Struktur sind allgemein Wirkstoffe zu verstehen, die als positiv geladene Ionen (Kationen) vorliegen oder die zur Bildung positiv positiv geladener Ionen in wässerigen Medien befähigt sind. Zum Beispiel haben viele biologisch aktive Mittel funktionelle Gruppen, die in wässrigem Medium jederzeit in ein positiv geladenes Ion und ein Gegenion dissoziieren, z.B. lösliche Salze basischer Wirkstoffe.

Der Ausdruck "Wirkstoffe" umfasst insbesondere therapeutisch aktive Mittel, pharmakologisch aktive Mittel und andere Mittel mit vorteilhaften Wirkungen, wenn man sie einem Menschen oder einem Tier verabreicht.

Im Allgemeinen bezeichnet der Begriff "Wirkstoffe" pharmazeutische Wirkstoffe oder Arzneimittel, also therapeutische Wirkstoffe. Der Ausdruck "Wirkstoffe" umfasst weiter auch Mittel zur Verwendung in der Veterinärmedizin.

Die vorliegende Erfindung ist besonders geeignet für die transdermale, insbesondere die iontophoretische, Verabreichung von Wirkstoffen wie

- Opioid-Agonisten, einschließlich Schmerzmittel wie Fentanyl, Sufentanyl, Morphin, Morphin-Derivate wie Codein oder die Heroin, Dihydrocodein, Hydromorphin, Oxycodon, Hydrocodon, Pethidin, Loperamid, Diphenoxylat, Methadon, Tramadol oder Tilidin;

- Opioid-Antagonisten wie Naloxon, Naltrexon;

- Gemischte Opiat-Agonisten/Antagonisten, wie Buprenorphin, Pentazocin, Nalbuphin;

- Antiemetika einschließlich $5$-$HT_3$-Rezeptor-Antagonisten wie Granisetron, Lerisetron, Ondansetron, Dolasetron, Metoclopramid sowie antidopaminerge Medikamente wie Domperidon, sowie H1-Rezeptor-Antagonisten wie beispielsweise Promethazin oder Meclozin sowie Muscarin-Antagonisten wie Scopolamin;

- Arzneimittelverbindungen, die auf das zentrale Nervensystem wirken, wie Rivastigmin, Galantamin, Tacrin, Donepezil, sowie Pramipexol, Adrenalin, Dopamin, Ropinirol, Nikotin, Fluphenazin, Chlorpromazin, Benzodiazepine, Monoamin-Wiederaufnahmehemmer wie Amitriptylin, Antidepressiva wie Mianserin;

- Alkaloide wie Ergotamin, Dihydroergotamin, Methysergid oder Lisurid, Belladonna-Alkaloide;

- Peptide, insbesondere Peptidhormone wie Insulin und Oxytocin oder Blutgerinnungsfaktoren und Wachstumshormone;

- kationisch aktive Indol-Verbindungen wie N-Dimethyltryptamin, Sumatriptan oder Psilocin;

- Lokalanästhetika wie Lidocain, Buprivacain, Artikain, Procain;

- Gastrointestinal wirkende Therapeutika, wie Carnitinchlorid oder Metoclopramid;

- Muskelrelaxantien wie Vancuroniumbromid;

- Antibiotika wie Tetracyclin, Tetracyclinbasierte-Zubereitungen, Kanamycin, Kanamycinbasierte-Zubereitungen, Gentamycin, Gentamycinbasierte-Zubereitungen oder Chinin;

- Gewichtsreduktionsmittel wie Fenfluramin oder Ephedrin;

- Antidiabetika wie Metformin;

- Inhibitoren der Thrombozytenaggregation, z. B. Ticlopidin oder Clopidogrel;

- Antiarrhythmika wie Chinidin oder Lidocain;

- Herz- oder Herz-Kreislaufmittel wie Dopamin, Noradrenalin, Methoxamin, Adrenalin, Verapamil, Diltiazem, Propranolol, Clonidin, Tolazolin;

- Sympathomimetika wie Salbutamol oder Terbutalin;

- Antihistaminika wie Clemastin, Cetirizin oder Chlorphenoxamin.

Die vorliegende Erfindung ist besonders geeignet für die transdermale, insbesondere die iontophoretische, Verabreichung eines Wirkstoffs aus der Gruppe der kationischen Indol-Verbindungen geeignet, besonders aus der Gruppe der kationischen Indol-Verbindungen, N-dimethyltryptamin und Psilocin, wobei die Gruppe auch die pharmazeutisch geeigneten Salze dieser kationisch aktiven Indol-Verbindungen mit umfasst.

Die vorstehend genannten kationischen Wirkstoffe können auch in Form von pharmazeutisch geeigneten Salzen vorliegen. Beispiele für pharmazeutisch geeignete Salze umfassen, Chlorid-, Bromid-, Jodid-, Schwefel-, Phosphat-, Lactat-, Citrat-, Tartrat-, Salicylat-, Succinat-, Maleat-, Gluconat-, Mesylat-, Laurat-, Dodecylat-, Myristat-, Palmitate-,Stearatsalze sind jedoch nicht beschränkt auf diese.

Die Intensität des Stroms während der Iontophorese sollte idealerweise den Wert von 600 $\mu$A /cm$^2$ nicht überschreiten, um das brennende Gefühl oder Verbrennungen der Haut zu vermeiden. Die Ausgangsspannung liegt im Allgemeinen im Bereich von 0,5 bis 10 V, je nach dem Widerstand zwischen den beiden Elektroden und dem Zwischenbereich der Haut, der normalerweise 50 k$\Omega$ oder mehr betragen kann.

In einer weiteren Ausführungsformen enthält das flüssigkeitsgetränkte Trägermaterial den kationischen Wirkstoff oder ein Salz davon in einer Menge im Bereich von 0,1 bis 20 Gew.-%, vorzugsweise von 0,2 bis 10 Gew.-%, besonders bevorzugt von 2 bis 10 Gew.-%, ganz besonders bevorzugt von 3 bis 5 Gew.-%, bezogen auf das Gesamtgewicht an Flüssigkeit in dem Trägermaterial.

Das flüssigkeitsgetränkte Trägermaterial gemäß der vorliegenden Erfindung enthält als Flüssigkeit vorzugsweise Wasser oder ein wässriges Lösungsmittelgemisch. Vorzugsweise ist der Anteil an Wasser in dem Lösungsmittelgemisch mindestens 15 Gew.-%, besonders bevorzugt mindestens 40 Gew.-%, bezogen auf das Gesamtgewicht der Flüssigkeit.

Gemäß einer weiteren Ausführungsform der Erfindung liegt der Wassergehalt oder der Anteil des Lösungsmittelgemisches im Bereich von 80 bis 99 Gew.-%.

Der Begriff "wässriges Lösemittelgemisch" umfasst im Allgemeinen flüssige Mischungen, die Wasser und mindestens ein weiteres Lösungsmittel enthalten, das im allgemeinen ein polares, mit Wasser mischbares organisches Lösungsmittel ist, wie z.B. Alkohole wie Ethanol, Isopropanol oder Glycerin.

Es gibt Anwendungen, bei denen ein kationischer Wirkstoff in Kombination mit mindestens einem weiteren Wirkstoff eingesetzt wird, der ausgewählt ist aus der Gruppe bestehend aus aktiven Mitteln mit einer neutralen Ladung und der sogar auch anionische Wirkstoffe umfassen kann.

Im Allgemeinen werden solche Wirkstoffe in dem System der vorliegenden Erfindung eingesetzt, die zur Durchdringung der Haut durch passive Diffusion befähigt sind oder die für die iontophoretische Hautpermeation geeignet sind.

In einer weiteren Ausführungsform kann das flüssigkeitsgetränkte Trägermaterial eine Hydrogel-Zusammensetzung sein, wobei zusätzliche gelbildende Polymere anwesend sind, die aus der Gruppe bestehend aus Polyacrylaten und Zellulosederivaten, wie Hydroxypropylmethylcellulose, Hydroxypropylcellulose oder Hydroxyethylcellulose, ausgewählt sein können.

Die Verwendung von Hydrogel-Zubereitungen bei der Iontophorese ist besonders vorteilhaft, weil in diesem Fall die Ionenstärke durch Veränderung des Anteils des Wassers innerhalb des Hydrogels eingestellt werden kann. Somit kann die Ionenstärke leicht eingestellt werden, um die Wirksamkeit des iontophoretischen Prozesses in jedem speziellen Fall zu optimieren.

In einer weiteren Ausführungsform weist die Flüssigkeit in dem flüssigkeitsgetränkten Trägermaterial einen pH-Wert im Bereich von 3 bis 8, vorzugsweise von 5,5 bis 7 besonders bevorzugt von etwa 6 auf.

Im Allgemeinen ist es bevorzugt, den pH-Wert so einzustellen, dass er sich nicht wesentlich von dem pH-Wert der Haut unterscheidet, wenn das TTS auf die Haut aufgebracht wird. In einer weiteren Ausführungsform ändert sich der pH-Wert der Haut zu $\pm$ 4,0 oder weniger, ungefähr $\pm$ 3,5 oder weniger, ungefähr $\pm$ 3,0 oder weniger, ungefähr $\pm$ 2,5 oder weniger, ungefähr $\pm$ 2,0 oder weniger, etwa $\pm$ 1,5 oder weniger, ungefähr $\pm$ 1,0 oder weniger oder etwa $\pm$ 0,5 oder weniger. Substanzen und Puffer für die Einstellung des pH-Wertes sind dem Fachmann bekannt.

Das flüssigkeitsgetränkte Trägermaterial kann optional weitere Additive enthalten, wobei die Additive ausgewählt sein können aus der Gruppe Lösungsvermittler, Hautdurchdringungsverstärker, Konservierungsmittel und antimikrobielle Mittel.

In diesem Zusammenhang bedeutet der Begriff "Löslichkeitsverbesserer" allgemein Verbindungen, die zur Erhöhung der Löslichkeit des kationisch aktiven Mittels in der Flüssigkeit beitragen können. Dies kann entweder durch Modulieren der möglichen Wechselwirkungen zwischen dem kationischen Wirkstoff und den anderen in der Flüssigkeit enthaltenen Komponenten oder durch den zusätzlichen Einbau geeigneter Hilfsstoffe erreicht werden.

Alternativ kann die Löslichkeit des Wirkstoffes durch Veränderung der Kristallmodifikation erreicht werden.

Beispiele für Lösungsvermittler umfassen Wasser, Diole, wie Propylenglykol und Glycerin, Monoalkohole wie Ethanol, Propanol und höhere Alkohole, Dimethylsulfoxid (DMSO), Dimethylformamid, N,N-Dimethylacetamid, N-substituierte Alkyl-Azacycloalkyl-2-one.

Ferner sind unter dem Begriff "Hautpermeationsverstärker" insbesondere Verbindungen zu verstehen, die eine Erhöhung der Permeabilität der Haut für einen Wirkstoff enthalten, insbesondere für einen kationischen Wirkstoff. Aufgrund dieser Erhöhung der Hautdurchlässigkeit wird die Rate, mit der der Wirkstoff die Haut durchdringt und in die Blutzirkulation eindringt, verstärkt.

Beispiele für Permeationsverstärker umfassen Dimethylsulfoxid (DMSO), N,N-Dimethylacetamid (DMA), Decylmethylsulfoxid (C10 MSO), Polyethylenglykolmonolaurat (PEGML), Propylenglykol (PG), Propylenglycolmonolaurat (PGML), Glycerinmonolaurat (GML), Lecithin, die 1-substituierten Alkyl-Azacycloalkyl-2-one, insbesondere Indodecylcylazacycloheptan-2-on, Alkohole und dergleichen.

Der Permeationsverstärker kann auch aus pflanzlichen Ölen ausgewählt werden, z.B. Safloröl, Baumwollsamenöl oder Maisöl.

Kombinationen, die zwei oder mehr verschiedene Permeationsverstärker enthalten, können ebenfalls verwendet werden.

Ferner sind unter dem Begriff "antimikrobielles Mittel" allgemein Mittel zu verstehen, die zur Verhinderung des Wachstums von Mikroben in einer pharmazeutischen Zubereitung, insbesondere in der Flüssigkeit des flüssigkeitsgetränkten Trägermaterials gemäß der vorliegenden Erfindung, geeignet sind.

Beispiele für geeignete antimikrobielle Mittel umfassen Salze von Chlorhexidin, wie Iodpropynyl Butylcarbamate, Diazolidinyl-Harnstoff, Chlorhexidindigluconat, Chlorhexidinacetat, Chlorhexidinisothionat oder Chlorhexidin-Hydrochlorid. Andere kationische antimikrobielle Mittel können ebenfalls verwendet werden, wie beispielsweise Benzalkoniumchlorid, Benzethoniumchlorid, Triclocarbon, Polyhexamethylenbiguanid, Cetylpyridiniumchlorid, Methylbenzethoniumchlorid.

Andere antimikrobielle Mittel umfassen halogenierte phenolische Verbindungen, wie 2,4, 4'-Trichlor-2-hydroxydiphenylether (Triclosan), parachlorometa Xylenol (PCMX), Methyl-para-hydroxybenzoat, und kurzkettige Alkohole, wie Ethanol, Propanol und dergleichen. Vorzugsweise ist die Gesamtkonzentration der antimikrobiellen Mittel im Bereich von 0,01 bis 2 Gew.-%, bezogen auf das Gesamtgewicht der Flüssigkeit, in der es enthalten ist.

Geeignete Trägermaterialien können Faserlagen, Gewebe, Gewirke, Schwämme, Schwammtuch, Gewirke-Vlies oder Gewebe-Filze oder filzartige Materialien usw. sein.

Die vorliegende Erfindung betrifft ferner die Verwendung des oben beschriebenen Systems als ein integraler Bestandteil eines iontophoretischen Pflasters, vorzugsweise als anodisches Reservoir des Pflasters.

Beispiel

Im Folgenden wird die Erfindung und ihre Wirksamkeit beispielhaft anhand der beigefügten Zeichnungen veranschaulicht.

Fig. 1 zeigt schematisch ein erfindungsgemäßes TTS in der Ansicht von unten, also von der Hautseite.

Fig. 2 zeigt schematisch ein erfindungsgemäßes TTS in der Ansicht von oben, also von der von der Haut abgewandten Seite.

Fig. 3a, 3b, 3c und 3d zeigen schematisch in Teilschritten wie das wirkstoffhaltige Trägermaterial aus seiner getrennt gelagerten Verpackung entnommen und an der Deckschicht des TTS fixiert wird.

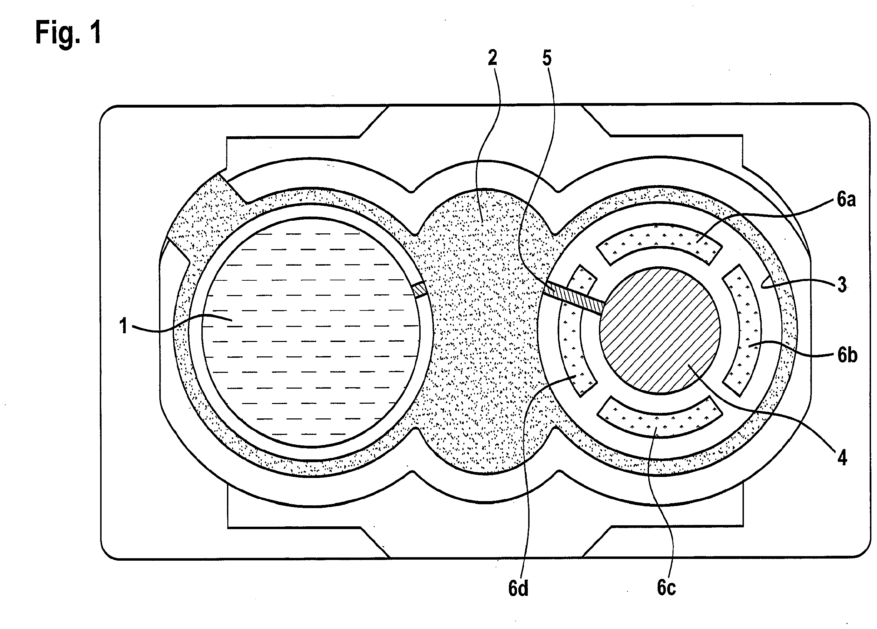

In Fig. 1 ist das wirkstoffhaltige Trägermaterial 1 zu erkennen, das eine runde Form besitzt uns das an der linken Fläche des TTS bereits in Position gebracht ist, während eine Abdeckfolie 2 mit einer Öffnung 3 an der rechten Seite den Blick auf die Elektrode 4, die elektrische Zuleitung 5 und vier gebogen geformte Klettbänder 6a, 6b, 6c und 6d ermöglicht. Auf die Klettbänder 6a, 6b, 6c und 6d wird dann im nächsten Schritt ein weiteres in der Abbildung nicht dargestelltes wirkstoffhaltiges Trägermaterial aufgepresst und mit den Klettbändern 6a, 6b, 6c und 6d ortsfest fixiert.

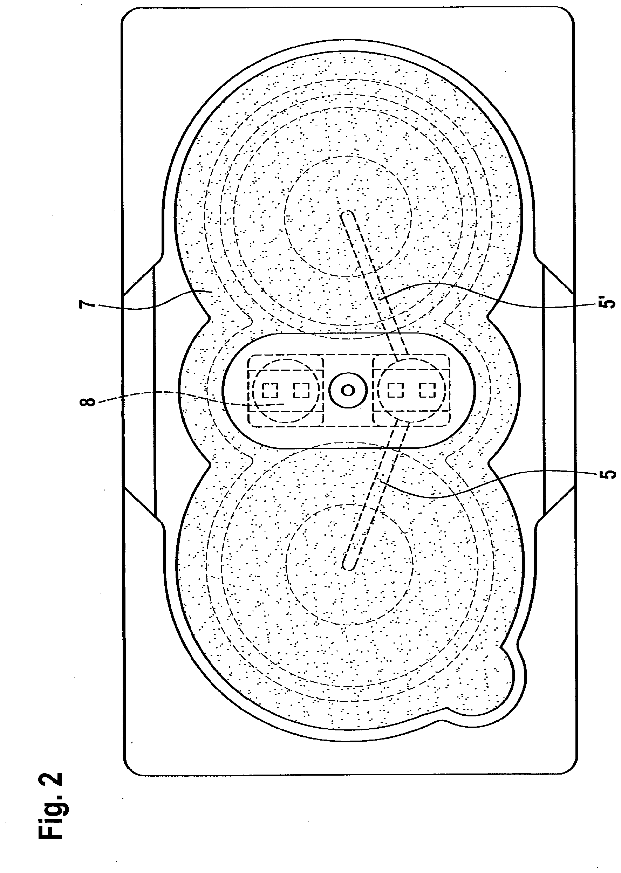

Fig. 2 zeigt im Wesentlichen die Deckschicht 7, durch die hindurch die linke Stromzufuhr 5 und die rechte Stromzufuhr 5' erkennbar sind. Mittig in der Darstellung ist die Einheit zur Stromversorgung 8 mit Batterie und elektronischer Regelung zu erkennen.

Fig. 3a zeigt im oberen Bereich die Deckschicht 7, an der zwei Klettbandsegmente 6a und 6c angeordnet sind, und im unteren Bereich das wirkstoffhaltige Trägermaterial 1 das in einer nach oben hin durch vorheriges, nicht dargestelltes Abziehen einer Verschlussfolie geöffneten Verpackung 10 lagert.

Fig. 3b zeigt wie das wirkstoffhaltige Trägermaterial 1 noch in der geöffneten Verpackung 10 und zusammen mit dieser an die Deckschicht 7 und die Klettbandsegmente 6a und 6c angenähert ist.

Fig. 3c zeigt wie das wirkstoffhaltige Trägermaterial 1 zusammen mit der Verpackung 10 von unten in Pfeilrichtung an die Klettbandsegmente 6a und 6c und an die Deckschicht 7 angepresst wird.

Fig. 3d zeigt, wie die Verpackung 10 nach unten entfernt wird, wobei das wirkstoffhaltige Trägermaterial 1 an den Klettbandsegmenten 6a und 6c und damit an der Deckschicht 7 fixiert bleibt.

**Patentansprüche**

1. Transdermales therapeutisches System (TTS) für die Abgabe von pharmazeutischen Wirkstoffen umfassend eine Deckschicht (7) und mindestens ein wirkstoffhaltiges Trägermaterial (1), wobei sich mindestens ein Halteelement zwischen wirkstoffhaltigem Trägermaterial und Deckschicht befindet, mit dem das wirkstoffhaltige Trägermaterial an der Deckschicht fixiert ist, **dadurch gekennzeichnet, dass** die therapeutischen Wirkstoffe eine kationische Struktur besitzen und das Trägermaterial flüssigkeitsgetränkt ist, das Halteelement die Hakenseite eines länglich geformten Segments eines Klettbandes ist und die Form des länglich geformten Klettbandes der Form des wirkstoffhaltigen Trägermaterials (1) angepasst ist.

2. TTS nach Anspruch 1, **dadurch gekennzeichnet, dass** die Befestigung des TTS auf der Haut über ein haftklebend ausgerüstetes Überpflaster erfolgt oder über eine flächig haftklebende oder musterförmig haftklebende Ausgestaltung der in Richtung zur Haut weisenden Seite der Deckschicht (7), wobei die Fläche oder die Flächen für das wirkstoffhaltiges Trägermaterial oder die wirkstoffhaltigen Trägermaterialien zumindest frei von Haftkleber sind.

3. TTS nach Anspruch 1 oder 2, **dadurch gekennzeichnet, dass** die Deckschicht (7) eine okklusive Deckschicht ist, die eine Barriereschicht für Flüssigkeiten und Wasserdampf ist.

4. TTS nach Anspruch 3, **dadurch gekennzeichnet, dass** die okklusive Deckschicht (7) aus einer Folie aus Kunststoff wie Polyethylen oder Polypropylen oder Polyester oder Polyurethan oder Polyamid besteht, und eine Dicke im Bereich von 5 bis 300 $\mu$m, vorzugsweise von 10 bis 200 $\mu$m, besonders bevorzugt von 12 bis 150 $\mu$m besitzt.

5. TTS nach einem oder nach mehreren der Ansprüche 1 bis 4, **dadurch gekennzeichnet, dass** das wirkstoffhaltige Trägermaterial (1) eine runde oder ovale Form besitzt und dass das länglich geformte Klettband die Form eines gebogenen Kreissegments hat mit einem Radius entsprechend dem Radius des runden oder ovalen flüssigkeitsgetränkten Trägermaterials.

6. TTS nach einem oder nach mehreren der Ansprüche 1 bis 5, **dadurch gekennzeichnet, dass** die Breite des länglich geformten Klettbandes variieren kann, wobei das Klettband nicht die gesamte Fläche der Deckschicht bedeckt, über die sich das wirkstoffhaltige Trägermaterial im TTS erstreckt.

7. TTS nach Anspruch 6, **dadurch gekennzeichnet, dass** das länglich geformte Klettband nur einen Teil der Fläche bedeckt, wobei es vorzugsweise in den seitenrandnahen Bereichen der Fläche angeordnet ist, über die sich das wirkstoffhaltige Trägermaterial im TTS erstreckt.

8. TTS nach einem oder nach mehreren der Ansprüche 1 bis 7, **dadurch gekennzeichnet, dass** es mehrere Klettbänder umfasst, die an gegenüberliegenden Seiten der Fläche angeordnet sind, über die sich das wirkstoffhaltige Trägermaterial im TTS erstreckt.

9. TTS nach einem oder nach mehreren der Ansprüche 1 bis 8, **dadurch gekennzeichnet, dass** das wirkstoffhaltige Trägermaterial (1) ein flüssigkeitsgetränktes Trägermaterial ist, wobei das Trägermaterial ein Vlies, ein textiles Material, ein Gewebe, ein Gewirke oder ein schwammförmiges Material oder Schwammtuch oder ein gelbildendes Polymer ist.

10. Verfahren zum Befestigen eines wirkstoffhaltigen Trägermaterials (1) an einer Deckschicht eines TTS in Anwesenheit von Klettbandsegmenten, **dadurch**

**gekennzeichnet, dass** das wirkstoffhaltige Trägermaterial flüssigkeitsgetränkt ist und in einer nach oben hin durch vorheriges Abziehen einer Verschlussfolie geöffneten Verpackung an die Deckschicht und die dort angeordneten Klettbandsegmente angenähert wird, wobei dann die Verpackung zusammen mit dem darin befindlichen wirkstoffhaltigen Trägermaterial durch Pressen an die Klettbandsegmente an der Deckschicht angedrückt wird, wobei danach die Verpackung losgelöst und entfernt wird und wobei das wirkstoffhaltige Trägermaterial an den Klettbandsegmenten und an der Deckschicht fixiert bleibt.

**Claims**

1. Transdermal therapeutic system (TTS) for the delivery of pharmaceutical actives, comprising a backing layer (7) and at least one active-containing carrier material (1), wherein at least one retaining element between the active-containing carrier material and the backing layer secures the active-containing carrier material to the backing layer, **characterized in that** the therapeutic actives have a cationic structure and the carrier material is liquid saturated, the retaining element is the hook side of an elongately shaped segment of a hook and loop tape and the shape of the elongately shaped hook and loop tape matches the shape of the active-containing carrier material (1).

2. TTS according to Claim 1, **characterized in that** the TTS is attached to the skin via a pressure-sensitively adhesive overplaster or via a uniformly or patternedly pressure-sensitively adhesive implementation of that side of the backing layer (7) which faces the skin, wherein the area(s) for the active-containing carrier material(s) are free from pressure-sensitive adhesive at least.

3. TTS according to Claim 1 or 2, **characterized in that** the backing layer (7) is an occlusive backing layer which is a barrier layer for liquids and water vapor.

4. TTS according to Claim 3, **characterized in that** the occlusive backing layer (7) consists of a self-supported film of a plastic such as polyethylene or polypropylene or polyester or polyurethane or polyamide and has a thickness in the range from 5 to 300 $\mu$m, preferably from 10 to 200 $\mu$m, more preferably from 12 to 150 $\mu$m.

5. TTS according to one or more of Claims 1 to 4, **characterized in that** the active-containing carrier material (1) has a round or oval shape and **in that** the elongately shaped hook and loop tape has the shape of an arcuate circular segment with a radius corresponding to the radius of the round or oval liquid-saturated carrier material.

6. TTS according to one or more of Claims 1 to 5, **characterized in that** the width of the elongately shaped hook and loop tape may vary, wherein the hook and loop tape does not cover the entire area of the backing layer whereover the active-containing carrier material in the TTS extends.

7. TTS according to Claim 6, **characterized in that** the elongately shaped hook and loop tape covers only a or one part of the area, preferably being disposed in the side edge near regions of the area whereover the active-containing carrier material in the TTS extends.

8. TTS according to one or more of Claims 1 to 7, **characterized in that** it comprises two or more hook and loop tapes arranged on opposite sides of the area whereover the active-containing carrier material in the TTS extends.

9. TTS according to one or more of Claims 1 to 8, **characterized in that** the active-containing carrier material (1) is a liquid-saturated carrier material, wherein the carrier material is a fibrous nonwoven web, a textile material, a woven fabric, a knitted fabric not produced by weft knitting with independently-movable needles, a sponge-shaped material, a sponge cloth or a gel-forming polymer.

10. Method of attaching an active-containing carrier material (1) to a TTS backing layer in the presence of hook and loop tape segments, **characterized in that** the active-containing carrier material is liquid saturated and **in that** the method comprises approximating the active-containing carrier material in a package upwardly opened by a closure film being removed beforehand to the backing layer and the hook and loop tape segments disposed thereon, then pressing the package together with the active-containing carrier material present therein against the hook and loop tape segments on the backing layer, thereafter releasing and removing the package and leaving the active-containing carrier material secured to the hook and loop tape segments and to the backing layer.

**Revendications**

1. Système thérapeutique transdermique (STT) pour la distribution de principes actifs pharmaceutiques, comprenant une couche de recouvrement (7) et au moins un matériau de support (1) contenant un principe actif, dans lequel au moins un élément de maintien se trouve entre le matériau de support contenant un principe actif et la couche de recouvrement, à l'aide duquel le matériau de support contenant un principe actif est fixé au niveau de la couche de recouvrement, **caractérisé en ce que** les principes actifs thérapeutiques possèdent une structure cationique, et le matériau de support est imprégné de liquide, l'élément de maintien est le côté à crochet d'un segment formé de manière allongée d'une bande Velcro et la forme de la bande Velcro formée de manière allongée est adaptée à la forme du matériau de support (1) contenant un principe actif.

2. STT selon la revendication 1, **caractérisé en ce que** la fixation du STT sur la peau est effectuée par l'intermédiaire d'un pansement couvrant autoadhésif ou par l'intermédiaire d'une configuration adhésive sur la surface ou adhésive de manière à présenter une forme de motif, du côté, pointant en direction de la peau, de la couche de recouvrement (7), dans lequel la surface ou les surfaces pour le matériau de support contenant un principe actif ou les matériaux de support contenant un principe actif sont au moins sans adhésif.

3. STT selon la revendication 1 ou 2, **caractérisé en ce que** la couche de recouvrement (7) est une couche de recouvrement occlusive, qui est une couche formant barrière pour des liquides ou de la vapeur d'eau.

4. STT selon la revendication 3, **caractérisé en ce que** la couche de recouvrement (7) occlusive est constituée d'un film en matière plastique telle que le polyéthylène ou le polypropylène ou le polyester ou le polyuréthane ou le polyamide et possède une épaisseur comprise dans la plage allant de 5 à 300 μm, de préférence allant de 10 à 200 μm, de manière particulièrement préférée allant de 12 à 150 μm.

5. STT selon l'une quelconque ou selon plusieurs des revendications 1 à 4, **caractérisé en ce que** le matériau de support (1) contenant un principe actif possède une forme ronde ou ovale, et que la bande Velcro formée de manière allongée a la forme d'un segment de cercle cintré présentant un rayon correspondant au rayon du matériau de support rond ou ovale imprégné de liquide.

6. STT selon l'une quelconque ou selon plusieurs des revendications 1 à 5, **caractérisé en ce que** la largeur de la bande Velcro formée de manière allongée peut varier, dans lequel la bande Velcro ne recouvre pas la totalité de la surface de la couche de recouvrement, sur laquelle le matériau de support contenant un principe actif s'étend dans le STT.

7. STT selon la revendication 6, **caractérisé en ce que** la bande Velcro formée de manière allongée ne recouvre qu'une partie de la surface, dans lequel le système est disposé de préférence dans les zones, proches du bord latéral, de la surface, sur laquelle le matériau de support contenant un principe actif s'étend dans le STT.

8. STT selon l'une quelconque ou selon plusieurs des revendications 1 à 7, **caractérisé en ce que** le STT comprend plusieurs bandes Velcro, qui sont disposées au niveau de côtés opposés de la surface, sur laquelle le matériau de support contenant un principe actif s'étend dans le STT.

9. STT selon l'une quelconque ou selon plusieurs des revendications 1 à 8, **caractérisé en ce que** le matériau de support (1) contenant un principe actif est un matériau de support imprégné de liquide, dans lequel le matériau de support est un non-tissé, un matériau textile, un tissu, un tricot ou un matériau spongieux ou un tissu-éponge ou un polymère formant un gel.

10. Procédé servant à fixer un matériau de support (1) contenant un principe actif au niveau d'une couche de recouvrement d'un STT en présence de segments de bande Velcro, **caractérisé en ce que** le matériau de support contenant un principe actif est imprégné de liquide et est rapproché de la couche de recouvrement et des segments de bande Velcro disposés à cet endroit dans un emballage ouvert vers le haut par un retrait préalable d'un opercule, dans lequel alors l'emballage est appliqué conjointement avec le matériau de support contenant un principe actif se trouvant dans l'emballage par compression au niveau des segments de bande Velcro au niveau de la couche de recouvrement, dans lequel par la suite l'emballage est détaché et est supprimé et dans lequel le matériau de support contenant un principe actif reste fixé au niveau des segments de bande Velcro et au niveau de la couche de recouvrement.

**Fig. 1**

Fig. 2

## Fig. 3a

## Fig. 3b

## Fig. 3c

## Fig. 3d

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5685837 A
- US 6745071 B
- WO 2012071175 A
- US 5558633 A
- US 20020019652 A1
- WO 2012071175 A1

**In der Beschreibung aufgeführte Nicht-Patentliteratur**

- **LUZARDO-ALVAREZ, A. et al.** Proceedings of the International Symposium on Controlled Release of bioactive Materials. 2000, 159-160